# EUROPEAN PATENT SPECIFICATION

(11) **EP 2 165 655 B1**
(45) Date of publication and mention of the grant of the patent: **20.04.2016**
(21) Application number: 09252215.0
(22) Date of filing: 17.09.2009
(51) Int. Cl.: A61B 17/29, A61B 17/072, A61B 17/00

(54) **SURGICAL INSTRUMENT WITH APPARATUS FOR MEASURING ELAPSED TIME BETWEEN ACTIONS**
Chirurgisches Instrument mit Vorrichtung zur Messung der verstrichenen Zeit zwischen Aktionen
Instrument chirurgical doté d'un appareil pour mesurer le temps écoulé entre les actions

(30) Priority: 18.09.2008 US 212951
(43) Date of publication of application: 24.03.2010
(73) Proprietor: ETHICON ENDO-SURGERY, INC., Cincinnati, Ohio 45242 (US)
(72) Inventor: Shelton, IV, Frederick E, Hillsboro, Ohio 45133 (US)
(74) Representative: Tunstall, Christopher Stephen

(56) References cited:
- EP-A- 1 813 199
- WO-A-2006/132992
- US-A1- 2006 278 680
- US-A1- 2008 078 807

## Description

### FIELD OF THE INVENTION

The present invention generally concerns surgical instruments with surgical implements that may perform multiple surgical procedures or actions and, more particularly, surgical cutting and fastening instruments with devices for measuring the elapsed time between steps in the surgical procedure.

### BACKGROUND OF THE INVENTION

Known surgical staplers include an end effector that simultaneously makes a longitudinal incision in tissue and applies lines of staples on opposing sides of the incision. The end effector includes a pair of cooperating jaw members that, if the instrument is intended for endoscopic or laparoscopic applications, are capable of passing through a cannula passageway. One of the jaw members receives a staple cartridge having at least two laterally spaced rows of staples. The other jaw member defines an anvil having staple-forming pockets aligned with the rows of staples in the cartridge. The instrument includes a plurality of reciprocating wedges which, when driven distally, pass through openings in the staple cartridge and engage drivers supporting the staples to effect the firing of the staples toward the anvil.

An example of a surgical stapler suitable for endoscopic applications is described in U.S. Pat. No. 5,465,895 which discloses an endocutter with distinct closing and firing actions. A clinician using this device is able to close the jaw members upon tissue to position the tissue prior to firing. Once the clinician has determined that the jaw members are properly gripping tissue, the clinician can then fire the surgical stapler with a single firing stroke, or multiple firing strokes, depending on the device. Firing the surgical stapler causes severing and stapling the tissue. The simultaneous severing and stapling avoids complications that may arise when performing such actions sequentially with different surgical tools that respectively only sever and staple.

One specific advantage of being able to close upon tissue before firing is that the clinician is able to verify via an endoscope that the desired location for the cut has been achieved, including a sufficient amount of tissue has been captured between opposing jaws. Otherwise, opposing jaws may be drawn too close together, especially pinching at their distal ends, and thus not effectively forming closed staples in the severed tissue. At the other extreme, an excessive amount of clamped tissue may cause binding and an incomplete firing.

Another problem that may be encountered during use of such instruments results when the clinician fails to allow the liquid in the tissue that is clamped in the end effector to drain out of the tissue before the tissue is cut and stapled. If the tissue is cut too quickly after it is clamped, the liquid therein may quickly traverse out of the tissue and impede formation of the staples.

The surgical stapler disclosed in WO 2006/132992 to Viola et al. purports to solve such problem by employing a controller that delays the firing of the staples until a predetermined amount of time has elapsed after clamping. A lead, switch or mechanical member may be employed to provide an audible or visual alert to inform the clinician that the preset period of time has elapsed for compression of tissue and that the firing can begin. If, however, the clinician desires to fire the device before the predetermined amount of time has lapsed, the stapler would not fire. Such inflexibility is undesirable.
US 2006/027868081 describes a surgical apparatus that has a clamp and a stapling mechanism. The stapler has a controller configured to control an actuation sled supported within a cartridge. The controller delays the actuation sleds movement to urge a plurality of staples from the cartridge for a pre-determined time period when an anvil is closed in position. The controller also controls an indicator to provide an indication when the pre-determined time period is reached.
EP 1813199 A1 describes a surgical instrument comprising a plurality of sensors and a status module. One of the sensors may be a knife position sensor. Further the status module may comprise a counter to determine the post clamping wait time.

Thus, there is a need for a surgical cutting and stapling device that is configured to enable the clinician to monitor the time that has lapsed between actions or steps in the surgical procedure, while still maintaining the ability to activate the instrument at any time.

There is a further need for a surgical cutting and stapling device that has the above-mentioned attributes such that the clinician can monitor the amount of time lapsed between actions or steps in the surgical procedure without looking away from the surgical site.

### SUMMARY

In a general aspect, the present invention is directed to a surgical cutting and fastening instrument comprising: a handle assembly; an end effector comprising: an elongate channel; a clamping member movably connected to the channel for selective movement between open and closed positions; and a knife movably supported within the elongate channel for selective travel therethrough; a closure system for selectively applying closing and opening motions to said clamping member; a drive system for selectively applying a drive motion to said movable knife to cause said knife to move from a proximal position to a distal position within said elongate channel; and a timing indicator on at least one of said end effector and said handle assembly to provide an indication of an amount of time that has elapsed after said clamping member has been moved to said closed position, while maintaining an ability of said drive system to selectively apply said drive motion to said movable knife, wherein said timing indicator, comprises: a timing device; and a series of at least two indicator lights electrically coupled to said timing device wherein one said indicator light is activated when said clamping member has been moved to said closed position and wherein another one of said indicator lights is activated after a first predetermined amount of time has lapsed after said clamping member has been moved to said closed position; the surgical cutting and fastening instrument further comprising: an encoder that interfaces with a control circuit to calculate the stage of deployment of the knife in the end effector, the control circuit sending signals to the lights to provide an indication of the location of the knife in the end effector.

### DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
Figures 1 and 2 are perspective views of a surgical cutting and fastening instrument according to various embodiments of the present invention;
Figures 3-5 are exploded views of an end effector and shaft of the instrument according to various embodiments of the present invention;
Figure 6 is a cross-sectional side view of an end effector according to various embodiments of the present invention;
Figure 7 is an exploded view of a handle assembly of the instrument according to various embodiments of the present invention;
Figures 8 and 9 are partial perspective views of a handle assembly according to various embodiments of the present invention;
Figure 10 is a side view of a portion of a handle assembly according to various embodiments of the present invention;
Figures 11 and 12 illustrate a proportional sensor that may be used according to various embodiments of the present invention;
Figure 13 is a schematic diagram of a circuit used in an instrument according to various embodiments of the present invention;
Figure 14 is a schematic diagram of another circuit used in an instrument according to various embodiments of the present invention;
Figure 15 is a perspective view of an end effector of various embodiments of the present invention;
FIG. 16 is a perspective view of another surgical instrument of various embodiments of the present invention; and
FIG. 17 is a perspective view of another surgical instrument of various embodiments of the present invention.

### DETAILED DESCRIPTION

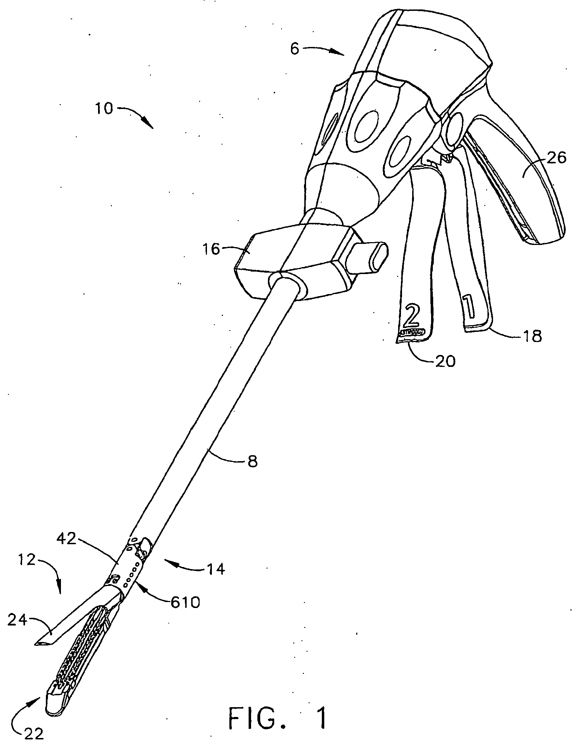

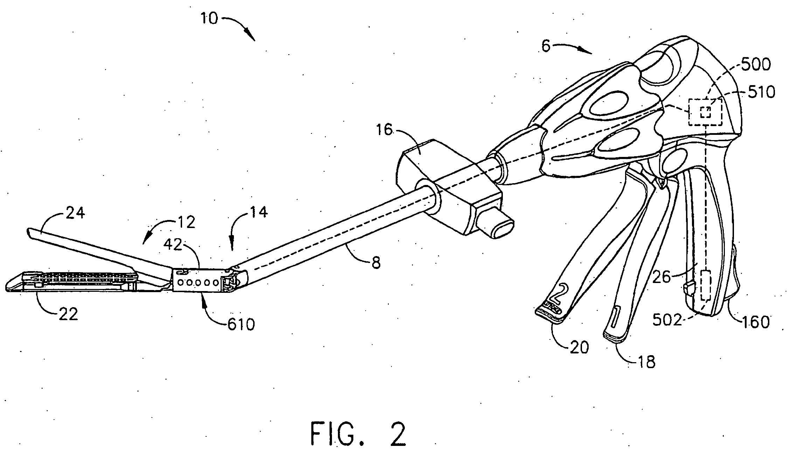

Figures 1 and 2 depict a surgical cutting and fastening instrument 10 according to various embodiments of the present invention. The illustrated embodiment is an endoscopic instrument and, in general, the embodiments of the instrument 10 described herein are endoscopic surgical cutting and fastening instruments. It should be noted, however, that according to other embodiments of the present invention, the instrument may be a non-endoscopic surgical instrument, such as a laparoscopic instrument. In addition, it will be further understood that other forms of surgical instruments are also contemplated.

The surgical instrument 10 depicted in Figures 1 and 2 comprises a handle assembly 6, a shaft 8, and an articulating surgical implement or end effector 12 pivotally connected to the shaft 8 at an articulation pivot 14. An articulation control 16 may be provided adjacent to the handle 6 to effect rotation of the end effector 12 about the articulation pivot 14. In the illustrated embodiment, the surgical implement or end effector 12 is configured to act as an endocutter for clamping, severing and stapling tissue. Although, in other embodiments, different types of surgical implements and end effectors may be used, such as graspers, cutters, staplers, clip appliers, access devices, drug/gene therapy devices, ultrasound, RF or laser devices, etc. wherein it may be desirable for the clinician to monitor the amount of time that has lapsed between activities or steps in the surgical procedure to be carried out by the instrument while still being able to control the instrument's various control systems.

The handle assembly 6 of the instrument 10 may include a closure trigger 18 and a firing trigger 20 for actuating the end effector 12. It will be appreciated that instruments having surgical implements or end effectors directed to different surgical tasks may have different numbers or types of triggers or other suitable controls for operating the end effector 12. The end effector 12 is shown separated from the handle assembly 6 by a preferably elongate shaft 8. In one embodiment, a clinician or operator of the instrument 10 may articulate the end effector 12 relative to the shaft 8 by utilizing the articulation control 16, as described in more detail in published U.S. Patent Application Publication No. US 2007/0158385 A1, filed January 10, 2006, entitled "Surgical Instrument Having An Articulating End Effector," by Geoffrey C. Hueil et al., . However, nonarticulatable devices are also contemplated and may effectively employ the unique and novel attributes of various embodiments of the present invention. Accordingly, the protection afforded to the various embodiments of the present invention should not be limited to articulatable instruments.

In this example, the end effector 12 includes, among other things, an elongate channel 22 configured to support a staple cartridge 34 therein. A pivotally translatable clamping member, such as an anvil 24, is movably supported on the elongate channel 22 at a spacing that assures effective stapling and severing of tissue clamped in the end effector 12. The handle assembly 6 may include a pistol grip 26 towards which a closure trigger 18 may be pivotally drawn by the clinician to cause clamping or closing of the anvil 24 toward a staple cartridge 34 to thereby clamp tissue positioned between the anvil 24 and staple cartridge 34. In this embodiment, the firing trigger 20 is farther outboard of the closure trigger 18. Once the closure trigger 18 is locked in the closed position, the firing trigger 20 may rotate slightly toward the pistol grip 26 so that it can be reached by the operator using one hand. Then the operator may pivotally draw the firing trigger 20 toward the pistol grip 12 to cause the stapling and severing of clamped tissue in the end effector 12. In other embodiments, different types of clamping members besides the anvil 24 could be used, such as, for example, an opposing jaw, etc.

It will be appreciated that the terms "proximal" and "distal" are used herein with reference to a clinician gripping the handle 6 of an instrument 10. Thus, the end effector 12 is distal with respect to the more proximal handle assembly 6. It will be further appreciated that, for convenience and clarity, spatial terms such as "vertical" and "horizontal" are used herein with respect to the drawings. However, surgical instruments are used in many orientations and positions, and these terms are not intended to be limiting and absolute.

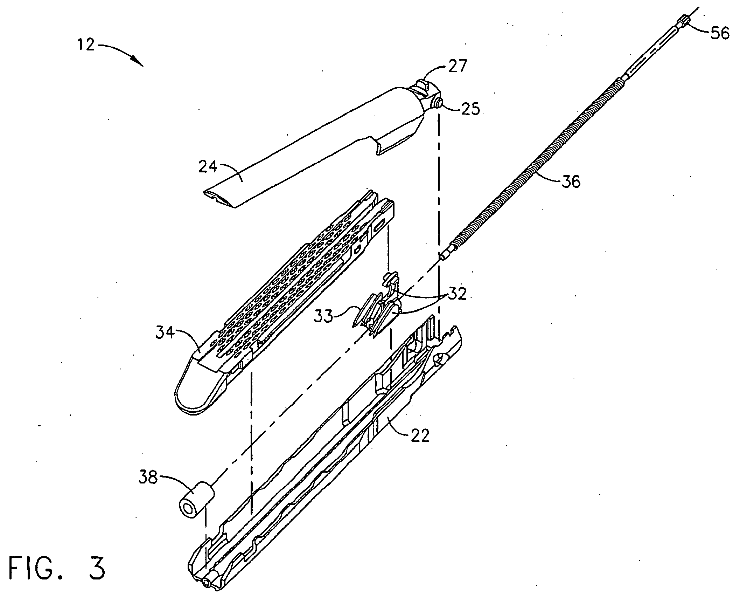

Figure 3 is an exploded view of the end effector 12 according to various embodiments of the present invention. As shown in the illustrated embodiment, the end effector 12 may include, in addition to the previously-mentioned channel 22 and anvil 24, a cutting instrument 32, a sled 33, a staple cartridge 34 that is removably seated in the channel 22, and a helical screw shaft 36. The cutting instrument 32 is a knife. The anvil 24 may be pivotably opened and closed at a pivot point 25 connected to the proximate end of the channel 22. The anvil 24 may also include a tab 27 at Its proximate end that is inserted into a component of the mechanical closure system (described further below) to open and close the anvil 24. When the closure trigger 18 is actuated, that is, drawn in by a user of the Instrument 10, the anvil 24 may pivot about the pivot point 25 into the clamped or closed position. If clamping of the end effector 12 is satisfactory, the operator may actuate the firing trigger 20, which, as explained in more detail below, causes the knife 32 and sled 33 to travel longitudinally along the channel 22, thereby cutting tissue clamped within the end effector 12. The movement of the sled 33 along the channel 22 causes the staples of the staple cartridge 34 to be driven through the severed tissue and against the closed anvil 24, which turns the staples to fasten the severed tissue. In various embodiments, the sled 33 may be an integral component of the cartridge 34. U.S. Pat. 6,978,921, entitled "Surgical Stapling Instrument Incorporating an E-beam Firing Mechanism," provides more details about such two-stroke cutting and fastening instruments. The sled 33 may be part of the cartridge 34, such that when the knife 32 retracts following the cutting operation, the sled 33 does not retract.

It should be noted that although the embodiments of the instrument 10 described herein employ an end effector 12 that staples the severed tissue, in other embodiments, different techniques for fastening or sealing the severed tissue may be used. For example, end effectors that use RF energy or adhesives to fasten the severed tissue may also be used. U.S. Pat. No. 5,709,680 entitled "Electrosurgical Hemostatic Device" to Yates et al., and U.S. Pat. No. 5,688,270 entitled "Electrosurgical Hemostatic Device With Recessed and/or Offset Electrodes" to Yates et at., disclose an endoscopic cutting instrument that uses RF energy to seal the severed tissue. U.S. Patent Application Serial No. 11/267,811 to Jerome R. Morgan, et. al, and U.S. Patent Application Serial No. 11/267,383 to Frederick E. Shelton, IV, et. al., disclose an endoscopic cutting instrument that uses adhesives to fasten the severed tissue. Accordingly, although the description herein refers to cutting/stapling operations and the like below, it should be recognized that this is an exemplary embodiment and is not meant to be limiting. The advantages provided by the various embodiments of the present invention may be equally attained in connection with other forms of surgical implements and end effectors.

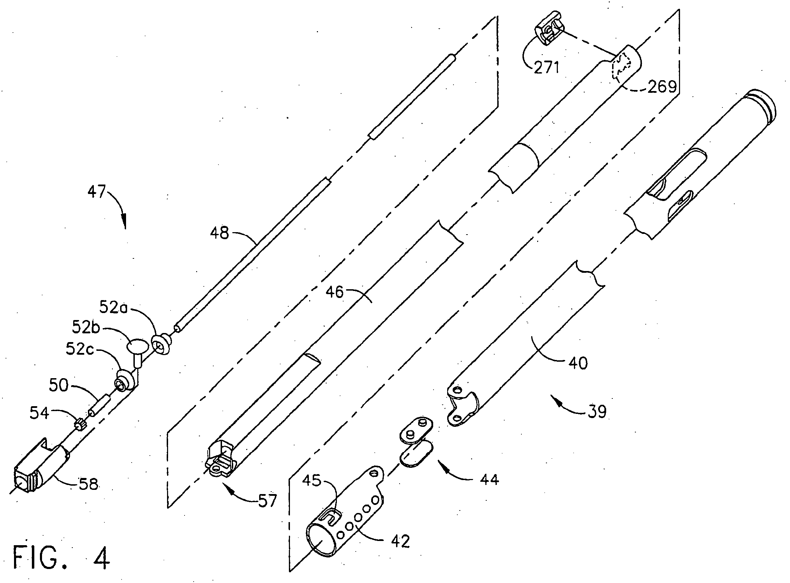

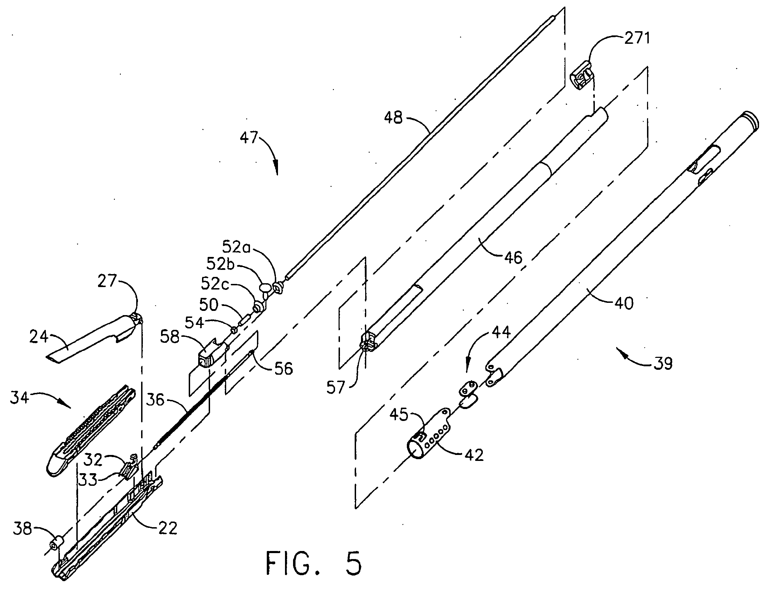

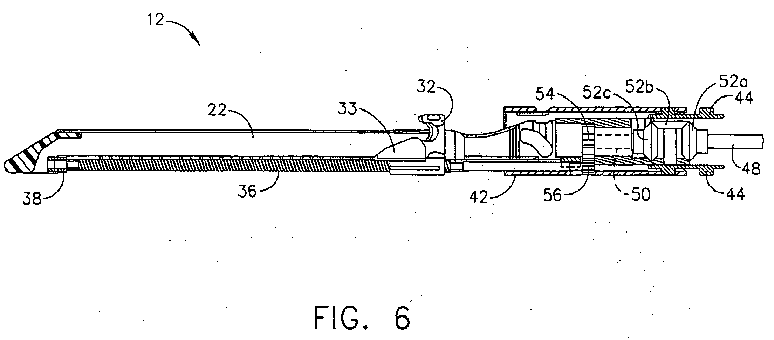

Figures 4 and 5 are exploded views and Figure 6 is a cross-sectional side view of the end effector 12 and shaft 8 according to various embodiments which illustrate one form of closure system 39 that may be employed to move the anvil 24 between open and closed positions. As shown in the illustrated embodiment, the closure system 39 may include a proximate closure tube 40 and a distal closure tube 42 pivotably linked by a pivot links 44. The distal closure tube 42 may include an opening 45 into which the tab 27 on the anvil 24 is inserted in order to open and close the anvil 24, as further described below. Disposed inside the closure tubes 40, 42 may be a proximate spine tube 46. Disposed inside the proximate spine tube 46 may be a portion of a drive system 47 that may, for example, comprise a main rotational (or proximate) drive shaft 48 that communicates with a secondary (or distal) drive shaft 50 via a bevel gear assembly 52. The secondary drive shaft 50 is connected to a drive gear 54 that engages a proximate drive gear 56 of the helical screw shaft 36. The vertical bevel gear 52b may be supported in an opening 57 in the distal end of the proximate spine tube 46. A distal spine tube 58 may be used to enclose the secondary drive shaft 50 and the drive gears 54, 56. Collectively, the main drive shaft 48, the secondary drive shaft 50, and the articulation assembly (e.g., the bevel gear assembly 52a-c) are sometimes referred to herein as the "main drive shaft assembly" which forms a portion of the drive system 47.

A bearing 38, positioned at a distal end of the staple channel 22, receives the helical drive screw 36, allowing the helical drive screw 36 to freely rotate with respect to the channel 22. See Figure 6. The helical screw shaft 36 may interface with a threaded opening (not shown) in the knife 32 such that rotation of the shaft 36 causes the knife 32 to translate distally or proximately (depending on the direction of the rotation) through the staple channel 22. Accordingly, when the main drive shaft 48 is caused to rotate by actuation of the firing trigger 20 (as explained in more detail below), the bevel gear assembly 52a-c causes the secondary drive shaft 50 to rotate, which in turn, because of the engagement of the drive gears 54, 56, causes the helical screw shaft 36 to rotate, which causes the knife driving member 32 to travel longitudinally along the channel 22 to cut any tissue clamped within the end effector. The sled 33 may be made of, for example, plastic, and may have a sloped distal surface. As the sled 33 traverse the channel 22, the sloped forward surface may push up or drive the staples in the staple cartridge through the clamped tissue and against the anvil 24. The anvil 24 forms the staples, thereby stapling the severed tissue. When the knife 32 is retracted, the knife 32 and sled 33 may become disengaged, thereby leaving the sled 33 at the distal end of the channel 22. Published U.S. Patent Application Publication No. US 2007/0233053 A1, entitled "Articulatable Drive Shaft Arrangements For Surgical Cutting and Fastening Instruments", filed May 30, 2007, discloses an exemplary embodiment of a motor-driven endocutter which may employ the unique and novel advantages of various embodiments of the present invention.

The closure system 39 and the drive system 47 may be referred to herein as "control systems" for applying "control motions" to various components of the surgical implement 12. Thus, the opening and closing motions applied by the closure system 39 are "control motions" as well as the firing motions applied by the drive system are "control motions".

Regardless of the type of surgical implement or end effector employed, many of the above-mentioned types of end effectors are used to perform more than one action during use. For example, the end effector 12 may be first used to grasp and manipulate tissue. Once the target tissue has been identified manipulated and positioned between the anvil and the staple cartridge, it is clamped therebetween by locking the closure trigger as described in the aforementioned U.S. Patent Publication No. US 2007/0233053 A1. As indicated above, it may be desirable to permit a predetermined amount of time, for example, approximately five-twenty seconds or more, to lapse before cutting through the tissue. Other end effectors may also be used to clamp or otherwise manipulate tissue prior to performing other actions on the tissue wherein it maybe desirable to permit a certain amount of time to lapse between such actions (even less than five seconds). Thus, while the various features and advantages of an embodiment of the present invention will now be explained with reference to the end effector 12 described above, the skilled artisan will readily understand that the various features of the present invention may find equal utility when employed with other forms of end effectors. Accordingly, the scope of protection afforded to various embodiments of the present invention should not be limited to the particular type of end effector specifically described herein.

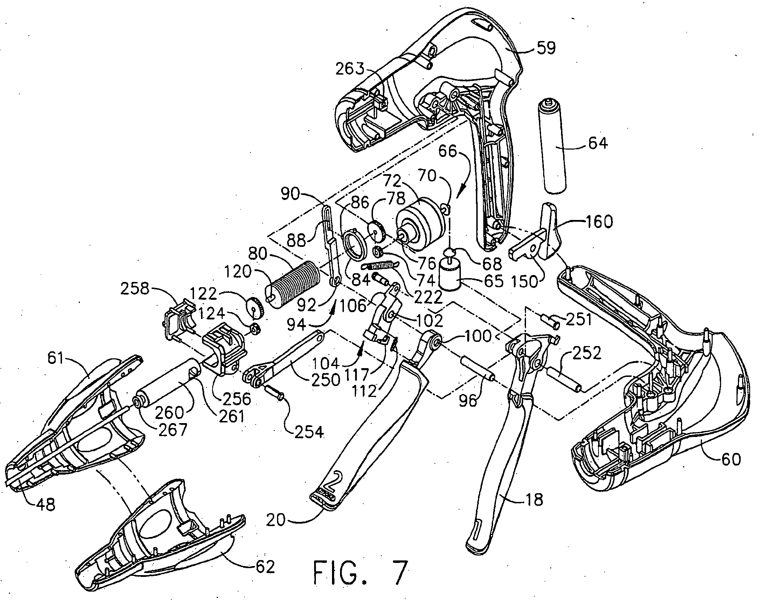

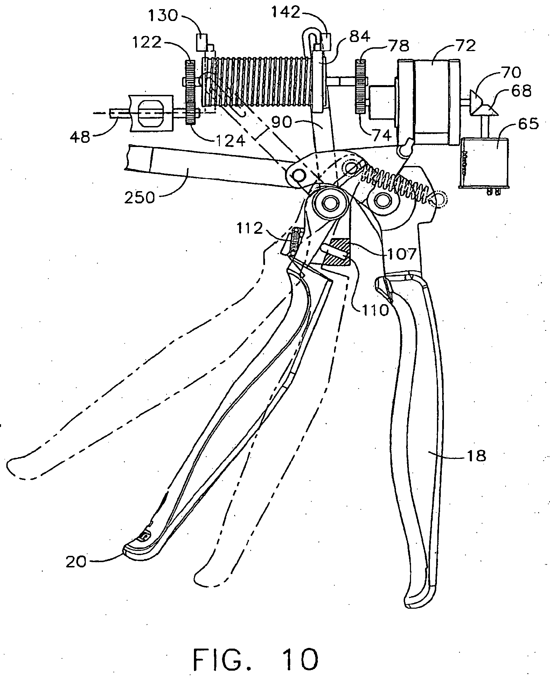

Figures 7-10 illustrate an exemplary embodiment of a motor-driven endocutter, and in particular the handle thereof, that provides user-feedback regarding the deployment and loading force of the cutting instrument in the end effector. In addition, the embodiment may use power provided by the user in retracting the firing trigger 20 to apply a "control motion" to the device (a so-called "power assist" mode). However, a variety of different endocutter drive arrangements could be employed. As shown in the illustrated embodiment, the handle 6 includes exterior lower side pieces 59, 60 and exterior upper side pieces 61, 62 that fit together to form, in general, the exterior of the handle assembly 6. A battery 64, such as a Li ion battery, may be provided in the pistol grip portion 26 of the handle assembly 6. The battery 64 powers, among other things, a motor 65 disposed in an upper portion of the pistol grip portion 26 of the handle assembly 6. According to various embodiments, the motor 65 may be a DC brushed driving motor having a maximum rotation of, approximately, 5000 RPM. The motor 64 may drive a 90° bevel gear assembly 66 comprising a first bevel gear 68 and a second bevel gear 70. The bevel gear assembly 66 may drive a planetary gear assembly 72. The planetary gear assembly 72 may include a pinion gear 74 connected to a drive shaft 76. The pinion gear 74 may drive a matting ring gear 78 that drives a helical gear drum 80 via a drive shaft 82. A ring 84 may be threaded on the helical gear drum 80. Thus, when the motor 65 rotates, the ring 84 is caused to travel along the helical gear drum 80 by means of the interposed bevel gear assembly 66, planetary gear assembly 72 and ring gear 78.

The handle assembly 6 may also include a run motor sensor 110 in communication with the firing trigger 20 to detect when the firing trigger 20 has been drawn in (or "closed") toward the pistol grip portion 26 of the handle assembly 6 by the operator to thereby actuate the cutting/stapling operation by the end effector 12. The sensor 110 may be a proportional sensor such as, for example, a rheostat or variable resistor. When the firing trigger 20 is drawn in, the sensor 110 detects the movement, and sends an electrical signal indicative of the voltage (or power) to be supplied to the motor 65. When the sensor 110 is a variable resistor or the like, the rotation of the motor 65 may be generally proportional to the amount of movement of the firing trigger 20. That is, if the operator only draws or closes the firing trigger 20 in a little bit, the rotation of the motor 65 is relatively low. When the firing trigger 20 is fully drawn in (or in the fully closed position), the rotation of the motor 65 is at its maximum. In other words, the harder the user pulls on the firing trigger 20, the more voltage is applied to the motor 65, causing greater rates of rotation.

The handle assembly 6 may include a middle handle piece 104 adjacent to the upper portion of the firing trigger 20. The handle 6 also may comprise a bias spring 112 connected between posts on the middle handle piece 104 and the firing trigger 20. The bias spring 112 may bias the firing trigger 20 to its fully open position. In that way, when the operator releases the firing trigger 20, the bias spring 112 will pull the firing trigger 20 to its open position, thereby removing actuation of the sensor 110, thereby stopping rotation of the motor 65. Moreover, by virtue of the bias spring 112, any time a user closes the firing trigger 20, the user will experience resistance to the closing operation, thereby providing the user with feedback as to the amount of rotation exerted by the motor 65. Further, the operator could stop retracting the firing trigger 20 to thereby remove force from the sensor 100, to thereby stop the motor 65. As such, the user may stop the deployment of the end effector 12, thereby providing a measure of control of the cutting/fastening operation to the operator.

The distal end of the helical gear drum 80 includes a distal drive shaft 120 that drives a ring gear 122, which mates with a pinion gear 124. The pinion gear 124 is connected to the main drive shaft 48 of the main drive shaft assembly. In that way, rotation of the motor 65 causes the main drive shaft assembly to rotate, which causes actuation of the end effector 12, as described above.

The ring 84 threaded on the helical gear drum 80 may include a post 86 that is disposed within a slot 88 of a slotted arm 90. The slotted arm 90 has an opening 92 its opposite end 94 that receives a pivot pin 96 that is connected between the handle exterior side pieces 59, 60. The pivot pin 96 is also disposed through an opening 100 in the firing trigger 20 and an opening 102 in the middle handle piece 104.

In addition, the handle assembly 6 may include a reverse motor (or end-of-stroke sensor) 130 and a stop motor (or beginning-of-stroke) sensor 142. In various embodiments, the reverse motor sensor 130 may be a limit switch located at the distal end of the helical gear drum 80 such that the ring 84 threaded on the helical gear drum 80 contacts and trips the reverse motor sensor 130 when the ring 84 reaches the distal end of the helical gear drum 80. The reverse motor sensor 130, when activated, sends a signal to the motor 65 to reverse its rotation direction, thereby withdrawing the knife 32 of the end effector 12 following the cutting operation.

The stop motor sensor 142 may be, for example, a normally-closed limit switch. In various embodiments, it may be located at the proximate end of the helical gear drum 80 so that the ring 84 trips the switch 142 when the ring 84 reaches the proximate end of the helical gear. drum 80.

In operation, when an operator of the instrument 10 pulls back the firing trigger 20, the sensor 110 detects the deployment of the firing trigger 20 and sends a signal to the motor 65 to cause forward rotation of the motor 65 at, for example, a rate proportional to how hard the operator pulls back the firing trigger 20. The forward rotation of the motor 65 in turn causes the ring gear 78 at the distal end of the planetary gear assembly 72 to rotate, thereby causing the helical gear drum 80 to rotate, causing the ring 84 threaded on the helical gear drum 80 to travel distally along the helical gear drum 80. The rotation of the helical gear drum 80 also drives the main drive shaft assembly as described above, which in turn applies a control motion (e.g., causes deployment of the knife 32 in the end effector 12). That is, the knife 32 and sled 33 are caused to traverse the channel 22 longitudinally, thereby cutting tissue clamped in the end effector 12. Also, the stapling operation of the end effector 12 is caused to happen in embodiments where a stapling-type end effector is used.

By the time the cutting/stapling operation of the end effector 12 is complete, the ring 84 on the helical gear drum 80 will have reached the distal end of the helical gear drum 80, thereby causing the reverse motor sensor 130 to be tripped, which sends a signal to the motor 65 to cause the motor 65 to reverse its rotation. This in turn causes the knife 32 to retract, and also causes the ring 84 on the helical gear drum 80 to move back to the proximate end of the helical gear drum 80.

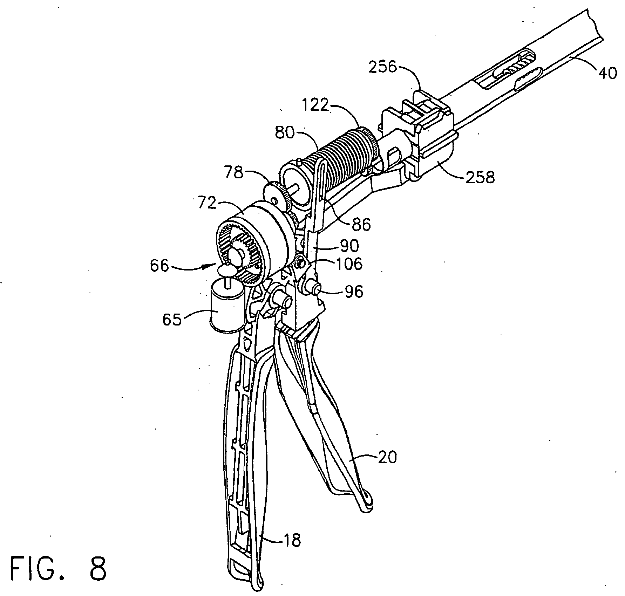

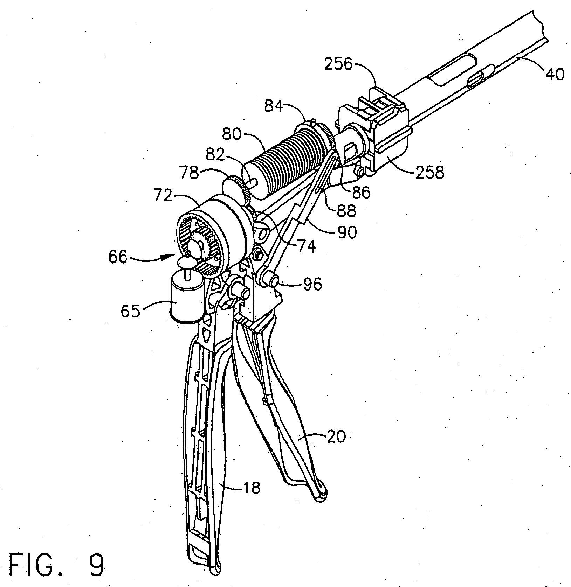

The middle handle piece 104 includes a backside shoulder 106 that engages the slotted arm 90 as best shown in Figures 8 and 9. The middle handle piece 104 also has a forward motion stop 107 that engages the firing trigger 20. The movement of the slotted arm 90 is controlled, as explained above, by rotation of the motor 65. When the slotted arm 90 rotates CCW as the ring 84 travels from the proximate end of the helical gear drum 80 to the distal end, the middle handle piece 104 will be free to rotate CCW. Thus, as the user draws in the firing trigger 20, the firing trigger 20 will engage the forward motion stop 107 of the middle handle piece 104, causing the middle handle piece 104 to rotate CCW. Due to the backside shoulder 106 engaging the slotted arm 90, however, the middle handle piece 104 will only be able to rotate CCW as far as the slotted arm 90 permits. In that way, if the motor 65 should stop rotating for some reason, the slotted arm 90 will stop rotating, and the user will not be able to further draw in the firing trigger 20 because the middle handle piece 104 will not be free to rotate CCW due to the slotted arm 90.

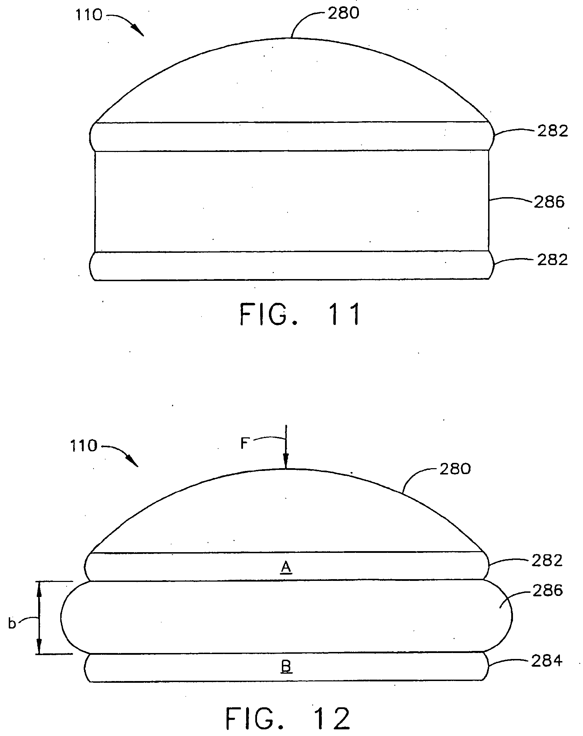

Figures 11 and 12 illustrate two states of a variable sensor that may be used as the run motor sensor 110 according to various embodiments of the present invention. The sensor 110 may include a face portion 280, a first electrode (A) 282, a second electrode (B) 284, and a compressible dielectric material 286 (e.g., EAP) between the electrodes 282, 284. The sensor 110 may be positioned such that the face portion 280 contacts the firing trigger 20 when retracted. Accordingly, when the firing trigger 20 is retracted, the dielectric material 286 is compressed, as shown in Figure 12, such that the electrodes 282, 284 are closer together. Since the distance "b" between the electrodes 282, 284 is directly related to the impedance between the electrodes 282, 284, the greater the distance the more impedance, and the closer the distance the less impedance. In that way, the amount that the dielectric 286 is compressed due to retraction of the firing trigger 20 (denoted as force "F" in Figure 12) is proportional to the impedance between the electrodes 282, 284, which can be used to proportionally control the motor 65.

Components of an exemplary closure system for applying another control motion (closing or clamping) the anvil 24 of the end effector 12 by retracting the closure trigger 18 are also shown in Figures 7-10. In the illustrated embodiment, the closure system includes a yoke 250 connected to the closure trigger 18 by a pin 251 that is inserted through aligned openings in both the closure trigger 18 and the yoke 250. A pivot pin 252, about which the closure trigger 18 pivots, is inserted through another opening in the closure trigger 18 which is offset from where the pin 251 is inserted through the closure trigger 18. Thus, retraction of the closure trigger 18 causes the upper part of the closure trigger 18, to which the yoke 250 is attached via the pin 251, to rotate CCW. The distal end of the yoke 250 is connected, via a pin 254, to a first closure bracket 256. The first closure bracket 256 connects to a second closure bracket 258. Collectively, the closure brackets 256, 258 define an opening in which the proximate end of the proximate closure tube 40 (see Figure 4) is seated and held such that longitudinal movement of the closure brackets 256, 258 causes longitudinal motion by the proximate closure tube 40. The instrument 10 also includes a closure rod 260 disposed inside the proximate closure tube 40. The closure rod 260 may include a window 261 into which a post 263 on one of the handle exterior pieces, such as exterior lower side piece 59 in the illustrated embodiment, is disposed to fixedly connect the closure rod 260 to the handle 6. In that way, the proximate closure tube 40 is capable of moving longitudinally relative to the closure rod 260. The closure rod 260 may also include a distal collar 267 that fits into a cavity 269 in proximate spine tube 46 and is retained therein by a cap 271 (see Figure 4).

In operation, when the yoke 250 rotates due to retraction of the closure trigger 18, the closure brackets 256, 258 cause the proximate closure tube 40 to move distally (i.e., away from the handle end of the instrument 10), which causes the distal closure tube 42 to move distally, which causes the anvil 24 to rotate about the pivot point 25 into the clamped or closed position. When the closure trigger 18 is unlocked from the locked position, the proximate closure tube 40 is caused to slide proximately, which causes the distal closure tube 42 to slide proximately, which, by virtue of the tab 27 being inserted in the window 45 of the distal closure tube 42, causes the anvil 24 to pivot about the pivot point 25 into the open or unclamped position. In that way, by retracting and locking the closure trigger 18, an operator may clamp tissue between the anvil 24 and channel 22, and may unclamp the tissue following the cutting/stapling operation by unlocking the closure trigger 20 from the locked position.

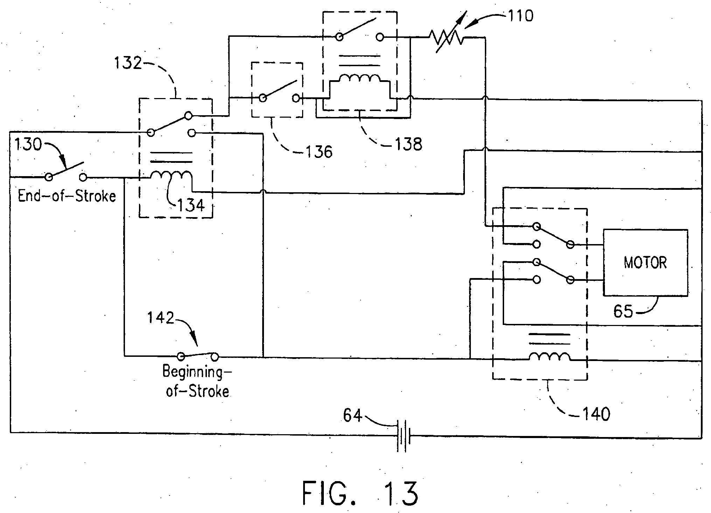

Figure 13 is a schematic diagram of an electrical circuit of the instrument 10 according to various embodiments of the present invention. When an operator initially pulls in the firing trigger 20 after locking the closure trigger 18, the sensor 110 is activated, allowing current to flow there through. If the normally-open reverse motor sensor switch 130 is open (meaning the end of the end effector stroke has not been reached), current will flow to a single pole, double throw relay 132. Since the reverse motor sensor switch 130 is not closed, the inductor 134 of the relay 132 will not be energized, so the relay 132 will be in its non-energized state. The circuit also includes a cartridge lockout sensor 136. If the end effector 12 includes a staple cartridge 34, the sensor 136 will be in the closed state, allowing current to flow. Otherwise, if the end effector 12 does not include a staple cartridge 34, the sensor 136 will be open, thereby preventing the battery 64 from powering the motor 65.

When the staple cartridge 34 is present, the sensor 136 is closed, which energizes a single pole, single throw relay 138. When the relay 138 is energized, current flows through the relay 136, through the variable resistor sensor 110, and to the motor 65 via a double pole, double throw relay 140, thereby powering the motor 65 and allowing it to rotate in the forward direction.

When the end effector 12 reaches the end of its stroke, the reverse motor sensor 130 will be activated, thereby closing the switch 130 and energizing the relay 134. This causes the relay 134 to assume its energized state (not shown in Figure 13), which causes current to bypass the cartridge lockout sensor 136 and variable resistor 110, and instead causes current to flow to both the normally-closed double pole, double throw relay 142 and back to the motor 65, but in a manner, via the relay 140, that causes the motor 65 to reverse its rotational direction.

Because the stop motor sensor switch 142 is normally-closed, current will flow back to the relay 134 to keep it closed until the switch 142 opens. When the knife 32 is fully retracted, the stop motor sensor switch 142 is activated, causing the switch 142 to open, thereby removing power from the motor 65.

In other embodiments, rather than a proportional-type sensor 110, an on-off type sensor could be used. In such embodiments, the rate of rotation of the motor 65 would not be proportional to the force applied by the operator. Rather, the motor 65 would generally rotate at a constant rate. But the operator would still experience force feedback because the firing trigger 20 is geared into the gear drive train.

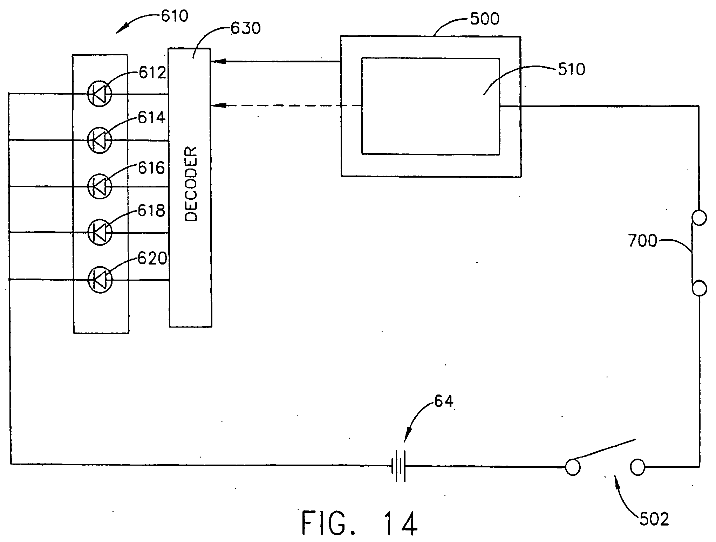

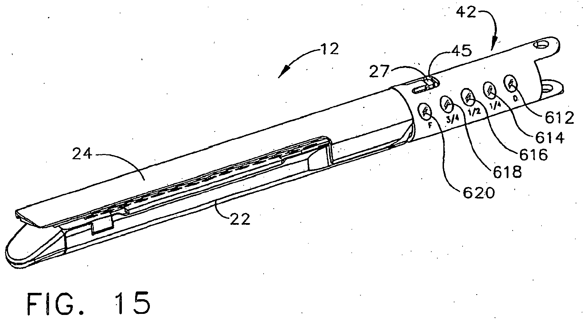

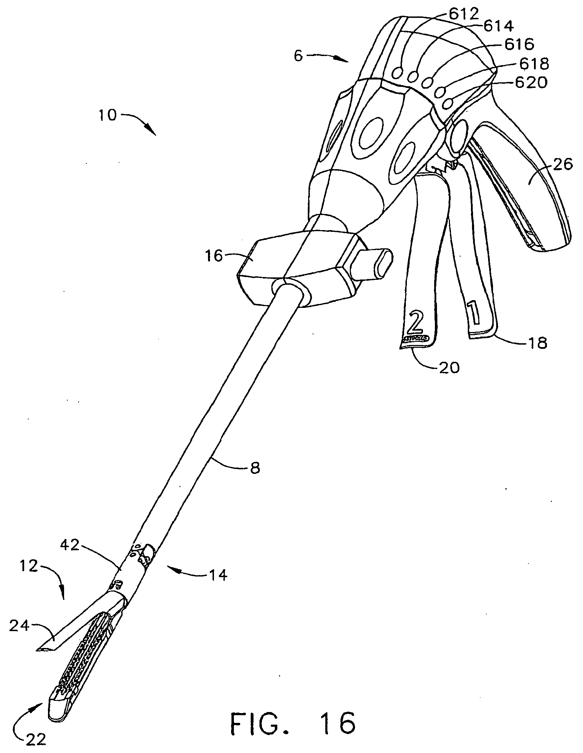

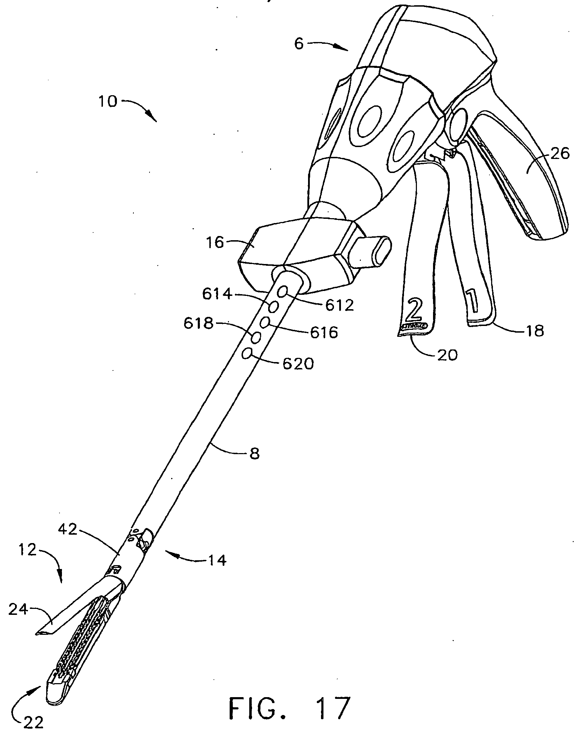

The instrument 10 may also include a control circuit, generally designated as 500, which may be implemented using a microcontroller or some other type of integrated circuit that may be employed as described in the aforementioned Patent Publication No. US 2007/0233053 A1. As can be seen in Figure 14, the control circuit 500 may be configured to receive a signal from a conventional closure trigger sensor 502 that may be supported within the handle portion 26 to detect when the closure trigger 18 has been locked in the closed position. The closure trigger sensor 502 may comprise, for example, a conventional limit switch that is normally open and is closed when the closure trigger 18 is locked in the closed position. However, other forms of sensors could be employed. The control circuit 500 may further have a timer component 510 that communicates with a series of indicator lights 610. Various numbers and arrangements of indicator lights may be employed. In the illustrated embodiment, for example, a first indicator light 612, a second indicator light 614, a third indicator light 616, a fourth indicator light 618 and a fifth indicator light 620 are employed. As can be seen in Figure 15, the indicator lights 612, 614, 616, 618, 620 may be located on the distal closure tube 42, so that the clinician can view them while viewing the end effector 12. In other embodiments, however, the lights 612, 614, 616, 618, 620 may be mounted on the handle assembly 6 (FIG. 16) or in the proximal end portion of the shaft 8 (FIG. 17). In various embodiments, the lights 612, 614, 616, 618, 620 may comprise light emitting diodes ("LED's"). The indicator lights 612, 614, 616, 618, 620 may be provided in the same color or different colors to assist the clinician in differentiating therebetween. As can also be seen in Figure 14, a conventional decoder 630 may be employed in connection with the control circuit 500 and the timer 510 to sequence the activation of the lights 612, 614, 616, 618, 620 in the manner described below.

When the clinician moves the closure trigger 18 to the fully closed and locked position, the first indicator light 612 may be powered. At that time, the timer component 510 begins the timing sequence. After a first predetermined amount of time has elapsed, for example, approximately five seconds, the controller 500 and decoder 630 powers the second indicator light 614. At that time, the first indicator light 612 may be de-energized or it may remain energized. The timer component 510 continues the timing sequence and, after a second predetermined amount of time has elapsed, for example, approximately an additional five seconds after the first predetermined amount of time has elapsed, the controller 500 and decoder 630 powers the third indicator light 616. At that time, the first and second indicator lights 612, 614 may remain energized or they may be de-energized. Thus, in this example, after approximately ten seconds has elapsed after the closure trigger 18 has been moved to the fully closed and locked position, the third indicator light 616 will be energized. The timer component 510 continues the timing sequence and, after a third predetermined amount of time (an additional five seconds), the controller 500/decoder 630 will power the fourth indicator light 618. At that time the first, second and third indicator lights 612, 614, 616 may remain powered or they may be de-energized. Thus, in this example, after approximately 15 seconds has elapsed after the closure trigger 18 has been moved to the fully closed and locked position, the fourth indicator light 618 will be energized. After the timer component 510 determines that a fourth predetermined amount of time has elapsed (an additional approximately five seconds), the controller 500/decoder 630 will power the fifth indicator light 620. At that time the first, second, third, and fourth indicator lights 612, 614, 616, 618, 620 may remain energized or they may be de-energized.

Thus, the clinician can ascertain approximately how much time has elapsed since the tissue was clamped in the end effector 12 by viewing the light indicators 612, 614, 616, 618, 620. If, during the process, the clinician desires to activate the drive system to cause the knife 32 and sled 33 to traverse the channel 22 before the entire time period has elapsed, he or she may do so by closing the firing trigger 20. In various embodiments, a second drive sensor 700 may be employed to detect when the firing trigger 20 has been drawn in or closed toward the pistol grip portion 26 of the handle assembly 6. As shown in Figure 14, in various embodiments, the second drive sensor 700 may comprise a "normally closed" switch such that when the firing trigger 20 is un-activated, the second drive sensor 700 remains in a closed position and when the firing trigger 20 is activated, the second drive sensor 700 is opened. When the second drive sensor is opened, the controller 500 resets the timer component 510 and all of the indicator lights 612, 614, 616, 618, 620 are de-energized. In various embodiments, to re-energize the indicator lights 612, 614, 616, 618, 620, the clinician would have to release the closure trigger 18 and then return it to the closed and locked position. In other embodiments the fifth indicator light 620 (and in other embodiments, all of the indicator lights 612, 614, 616, 618, 620) would remain energized until the closure trigger 18 was moved to the unlocked position to release the clamped tissue. In still other embodiments, when the second drive sensor 700 is opened, the timer component 510 may begin recounting the amount of time that has elapsed from the activation to the drive system to enable the clinician to monitor the duration of the firing sequence. Again, the control circuit decoder 630 may control the lighting sequence of the indicator lights 612, 614, 616, 618, 620 as was described above in five second intervals or other time intervals if desired. In still other embodiments, the timing component 510 may begin recounting when the application of the drive motion has been discontinued or interrupted to provide the clinician with an indication of the amount of time that has elapsed since the drive motion was discontinued.

Other embodiments may employ different drive system arrangements for applying various control motions and/or different sensor arrangements. The present invention employs an encoder that interfaces with the control circuit 500 to calculate the stage of deployment of the knife 32 in the end effector 12. That is, the control circuit can calculate If the knife 32 is fully deployed, fully retracted, or at an intermittent stage. the controller 500 sends signals to the lights 612, 614, 616, 618, 620 to provide the clinician of an indication of the location of the knife 32 in the end effector as it is traverses from the proximal end of the elongate channel 22 to the distal end thereof.

While the above-described embodiment employs a control circuit or controller that has a conventional timing component or system, other conventional timer arrangements could be employed without departing from the scope of the present invention. The embodiments depicted in Figures 1, 2 and 15, illustrate use of the indicator lights 612, 614, 616, 618, 620 on the distal tube segment 42. Such arrangement permits the clinician to view the lights when viewing the end effector 12 within the surgical site. Therefore, the clinician does not have to look away from the surgical site to ascertain how much time has transpired between actions. In the embodiment depicted in FIG. 16, the indicator lights 612, 614, 616, 618, 620 are mounted in the handle assembly 6 and in the instrument 10" depicted in FIG. 17, the indicator lights 612, 614, 616, 618, 620 are mounted in the proximal end of the shaft 8.

The end effector 12 described herein is particularly suited to clamp and manipulate tissue as well as cut and sever it. However the indicator light arrangements and their equivalent structures may be effectively used in connection with a variety of different end effectors and surgical implements wherein the implement is used to perform multiple "actions" and where it is desirable for the clinician to know how much time has elapsed after commencing an action while maintaining the ability to activate the surgical instrument. For example, the surgical implement could be a non-cutting, non-stapling endoscopic instrument such as a grasper, a stapler, a clip applier, an access device, a drug/gene therapy delivery device, an energy device using ultrasound, RF, laser, etc.

The devices disclosed herein can be designed to be disposed of after a single use, or they can be designed to be used multiple times. In either case, however, the device may be reconditioned for reuse after at least one use. Reconditioning may include any combination of the steps of disassembly of the device, followed by cleaning or replacement of particular pieces, and subsequent reassembly. In particular, the device may be disassembled, and any number of particular pieces or parts of the device can be selectively replaced or removed in any combination. Upon cleaning and/or replacement of particular parts, the device may be reassembled for subsequent use either at a reconditioning facility, or by a surgical team immediately prior to a surgical procedure. Those of ordinary skill in the art will appreciate that the reconditioning of a device may utilize a variety of different techniques for disassembly, cleaning/replacement, and reassembly. Use of such techniques, and the resulting reconditioned device, are all within the scope of the present application.

Preferably, the invention described herein will be processed before surgery. First a new or used instrument is obtained and, if necessary, cleaned. The instrument can then be sterilized. In one sterilization technique, the instrument is placed in a closed and sealed container, such as a plastic or TYVEK® bag. The container and instrument are then placed in a field of radiation that can penetrate the container, such as gamma radiation, x-rays, or higher energy electrons. The radiation kills bacteria on the instrument and in the container. The sterilized instrument can then be stored in the sterile container. The sealed container keeps the instrument sterile until it is opened in the medical facility.

The invention which is intended to be protected is not to be construed as limited to the particular embodiments disclosed. The embodiments are therefore to be regarded as illustrative rather than restrictive. Accordingly, it is expressly intended that all such equivalents, variations and changes which fall within the scope of the present invention as defined in the claims be embraced thereby.

## Claims

1. A surgical cutting and fastening instrument (10) comprising:
a handle assembly (6);
an end effector (12) comprising:
an elongate channel (22);
a clamping member (24) movably connected to the channel (22) for selective movement between open and closed positions; and
a knife (32) movably supported within the elongate channel (22) for selective travel therethrough;
a closure system for selectively applying closing and opening motions to said clamping member (24);
a drive system for selectively applying a drive motion to said movable knife (32) to cause said knife (32) to move from a proximal position to a distal position within said elongate channel (22); and
a timing indicator (610) on at least one of said end effector (12) and said handle assembly (6) to provide an indication of an amount of time that has elapsed after said clamping member (24) has been moved to said closed position, while maintaining an ability of said drive system to selectively apply said drive motion to said movable knife (32), wherein said timing indicator (610), comprises:
a timing device (510); and
a series of at least two indicator lights (612, 614) electrically coupled to said timing device (510) wherein one said indicator light (612) is activated when said clamping member (24) has been moved to said closed position and wherein another one of said indicator lights (614) is activated after a first predetermined amount of time has lapsed after said clamping member (24) has been moved to said closed position;
the surgical cutting and fastening instrument (10) further comprising:
an encoder that interfaces with a control circuit (500) to calculate the stage of deployment of the knife (32) in the end effector (12), the control circuit configured to send signals to the lights (610) to provide an indication of the location of the knife (32) in the end effector (12).

2. The surgical cutting and fastening instrument (10) of claim 1 wherein said timing indicator (510) begins recounting after each said surgical action.

3. The surgical cutting and fastening instrument (10) of claim 1 wherein said series of indicator lights (610) comprises at least three indicator lights (612, 614, 616) wherein a third said indicator light (616) is activated after a second predetermined amount of time has elapsed after the activation of said another one of said indicator lights (612, 614).

4. The surgical cutting and fastening instrument (10) of claim 3 wherein said series of indicator lights (610) comprises at least four indicator lights (612, 614, 616, 618) wherein a fourth indicator light (618) is activated after a third predetermined amount of time has passed after the activation of said third indicator light (612, 614, 616).

5. The surgical cutting and fastening instrument (10) of claim 4 wherein said series of indicator lights (610) comprises at least five indicator lights (612, 614, 616, 618, 620) wherein a fifth indicator light (620) is activated after a fourth predetermined amount of time has passed after the activation of said fourth indicator light (618).

6. The surgical cutting and fastening instrument (10) of claim 5 wherein said first, second, third and fourth predetermined amounts of time each comprise approximately five seconds.

7. The surgical cutting and fastening instrument (10) of claim 1 wherein said timing device (510) is supported in said handle assembly (6).

8. The surgical cutting and fastening instrument (10) of claim 1 wherein each said indicator light (610) comprises a light emitting diode.

9. The surgical cutting and fastening instrument (10) of claim 1 wherein said timing indicator (510) begins recounting upon application of said drive motion.

10. The surgical cutting and fastening instrument (10) of claim 9 wherein said timing indicator (510) begins recounting when said application of said drive motion has been discontinued.

11. A method for processing a surgical cutting and fastening implement, the method comprising:
obtaining the surgical cutting and fastening implement (10) of any preceding claim;
sterilizing at least part of it; and
storing the sterile part in a sterile container.

## Patentansprüche

1. Chirurgisches Schneide- und Befestigungsinstrument (10), umfassend:
eine Griffanordnung (6);
ein Greiforgan (12), umfassend:
einen langgestreckten Kanal (22);
ein Klemmelement (24), das zur selektiven Bewegung zwischen offenen und geschlossenen Stellungen beweglich mit dem Kanal (22) verbunden ist; und
ein Messer (32), das beweglich in dem langgestreckten Kanal (22) zur selektiven Bewegung durch ihn hindurch unterstützt wird;
ein Verschlusssystem zum selektiven Aufbringen von Schließ- und Öffnungsbewegungen auf das Klemmelement (24) ;
ein Antriebssystem zum selektiven Aufbringen einer Antriebsbewegung auf das bewegliche Messer (32), damit sich das Messer (32) aus einer proximalen Stellung in eine distale Stellung in dem langgestreckten Kanal (22) bewegt; und
eine Zeitanzeige (610) wenigstens auf dem Greiforgan (12) und/oder auf der Griffanordnung (6) zur Bereitstellung eines Hinweises über eine Zeitdauer, die verstrichen ist, nachdem das Klemmelement (24) in die geschlossene Stellung bewegt wurde, unter Beibehaltung einer Fähigkeit des Antriebssystems, die Antriebsbewegung selektiv auf das bewegliche Messer (32) aufzubringen, wobei die Zeitanzeige (610) Folgendes umfasst:
ein Zeitmessgerät (510); und
eine Reihe von wenigstens zwei Anzeigelampen (612, 614), die elektrisch mit dem Zeitmessgerät (510) verbunden sind, wobei eine dieser Anzeigelampen (612) aktiviert wird, wenn das Klemmelement (24) in die geschlossene Stellung bewegt wurde, und wobei eine andere der Anzeigelampen (614) aktiviert wird, nachdem eine erste vorbestimmte Zeitdauer nach Bewegung des Klemmelements (24) in die geschlossene Stellung verstrichen ist;
wobei das chirurgische Schneide- und Befestigungsinstrument (10) ferner Folgendes umfasst:
ein Kodiergerät, das mit einem Steuerkreislauf (500) verbunden ist, um das Stadium des Einsatzes des Messers (32) in dem Greiforgan (12) zu berechnen, wobei der Steuerkreislauf ausgebildet ist, um Signale an die Lampen (610) zu senden, um einen Hinweis auf die Position des Messers (32) in dem Greiforgan (12) zu liefern.

2. Chirurgisches Schneide- und Befestigungsinstrument (10) nach Anspruch 1, wobei die Zeitanzeige (510) nach jeder chirurgischen Aktion erneut zu zählen beginnt.

3. Chirurgisches Schneide- und Befestigungsinstrument (10) nach Anspruch 1, wobei die Reihe von Anzeigelampen (610) wenigstens drei Anzeigelampen (612, 614, 616) umfasst, wobei eine dritte Anzeigelampe (616) aktiviert wird, nachdem eine zweite vorbestimmte Zeitdauer nach Aktivierung einer anderen der Anzeigelampen (612, 614) verstrichen ist.

4. Chirurgisches Schneide- und Befestigungsinstrument (10) nach Anspruch 3, wobei die Reihe von Anzeigelampen (610) wenigstens vier Anzeigelampen (612, 614, 616, 618) umfasst, wobei eine vierte Anzeigelampe (618) aktiviert wird, nachdem eine dritte vorbestimmte Zeitdauer nach Aktivierung der dritten Anzeigelampe (612, 614, 616) verstrichen ist.

5. Chirurgisches Schneide- und Befestigungsinstrument (10) nach Anspruch 4, wobei die Reihe von Anzeigelampen (610) wenigstens fünf Anzeigelampen (612, 614, 616, 618, 620) umfasst, wobei eine fünfte Anzeigelampe (620) aktiviert wird, nachdem eine vierte vorbestimmte Zeitdauer nach Aktivierung der vierten Anzeigelampe (618) verstrichen ist.

6. Chirurgisches Schneide- und Befestigungsinstrument (10) nach Anspruch 5, wobei die ersten, zweiten, dritten und vierten vorbestimmten Zeitdauern jeweils ungefähr fünf Sekunden umfassen.

7. Chirurgisches Schneide- und Befestigungsinstrument (10) nach Anspruch 1, wobei das Zeitmessgerät (510) in der Griffanordnung (6) unterstützt wird.

8. Chirurgisches Schneide- und Befestigungsinstrument (10) nach Anspruch 1, wobei jede Anzeigelampe (610) eine Leuchtdiode umfasst.

9. Chirurgisches Schneide- und Befestigungsinstrument (10) nach Anspruch 1, wobei die Zeitanzeige (510) bei Aufbringen einer Antriebsbewegung erneut zu zählen beginnt.

10. Chirurgisches Schneide- und Befestigungsinstrument (10) nach Anspruch 9, wobei die Zeitanzeige (510) erneut zu zählen beginnt, wenn keine Antriebsbewegung mehr aufgebracht wird.

11. Verfahren zur Aufbereitung eines chirurgischen Schneide- und Befestigungsinstruments, wobei das Verfahren Folgendes umfasst:
Erhalten des chirurgischen Schneide- und Befestigungsinstrument (10) nach einem der vorhergehenden Ansprüche;
Sterilisieren zumindest eines Teils davon; und
Aufbewahren des sterilen Teils in einem sterilen Behälter.

## Revendications

1. Instrument chirurgical (10) de coupe et de fixation, comprenant :
un ensemble poignée (6) ;
un effecteur terminal (12) comprenant :
un canal allongé (22) ;
un élément de serrage (24) raccordé mobile au canal (22) pour se déplacer de façon sélective entre des positions ouverte et fermée ; et
un couteau (32) soutenu mobile à l'intérieur du canal allongé (22) pour se déplacer de façon sélective à travers celui-ci ;
un système de fermeture pour appliquer sélectivement des mouvements d'ouverture et de fermeture audit élément de serrage (24) ;
un système d'entraînement pour appliquer sélectivement un mouvement d'entraînement audit couteau (32) mobile afin de faire se déplacer ledit couteau (32) d'une position proximale à une position distale à l'intérieur dudit canal allongé (22) ; et
un indicateur de durée (610) sur ledit effecteur terminal (12) et/ou ledit ensemble poignée (6) pour fournir une indication du temps qui s'est écoulé après que ledit élément de serrage (24) a été déplacé à ladite position fermée, tout en maintenant la capacité dudit système d'entraînement à appliquer sélectivement ledit mouvement d'entraînement audit couteau (32) mobile, dans lequel ledit indicateur de durée (610) comprend :
un dispositif de chronométrage (510) ; et
une série d'au moins deux voyants lumineux (612, 614) couplés électriquement audit dispositif de chronométrage (510), un (612) desdits voyants lumineux étant activé quand ledit élément de serrage (24) a été déplacé vers ladite position fermée et un autre (614) desdits voyants lumineux étant activé après que se soit écoulée une première durée prédéterminée après que ledit élément de serrage (24) ait été déplacé vers ladite position fermée ;
l'instrument chirurgical (10) de coupe et de fixation comprenant en outre :
un encodeur qui interface avec un circuit de commande (500) pour calculer l'étape de déploiement du couteau (32) dans l'effecteur terminal (12), le circuit de commande étant configuré pour envoyer des signaux aux voyants (610) afin de fournir une indication de l'emplacement du couteau (32) dans l'effecteur terminal (12).

2. Instrument chirurgical (10) de coupe et de fixation selon la revendication 1, dans lequel ledit indicateur de durée (510) recommence à compter après chacune desdites actions chirurgicales.

3. Instrument chirurgical (10) de coupe et de fixation selon la revendication 1, dans lequel ladite série de voyants lumineux (610) comprend au moins trois voyants lumineux (612, 614, 616) parmi lesquels ledit troisième voyant lumineux (616) est activé après que se soit écoulée une deuxième durée prédéterminée après l'activation d'un autre desdits voyants lumineux (612, 614).

4. Instrument chirurgical (10) de coupe et de fixation selon la revendication 3, dans lequel ladite série de voyants lumineux (610) comprend au moins quatre voyants lumineux (612, 614, 616, 618) parmi lesquels un quatrième voyant lumineux (618) est activé après que se soit écoulée une troisième durée prédéterminée après l'activation dudit troisième voyant lumineux (612, 614, 616).

5. Instrument chirurgical (10) de coupe et de fixation selon la revendication 4, dans lequel ladite série de voyants lumineux (610) comprend au moins cinq voyants lumineux (612, 614, 616, 618, 620) parmi lesquels un cinquième voyant lumineux (620) est activé après que se soit écoulée une quatrième durée prédéterminée après l'activation dudit quatrième voyant lumineux (618).

6. Instrument chirurgical (10) de coupe et de fixation selon la revendication 5, dans lequel lesdites première, deuxième, troisième et quatrième durées prédéterminées font chacune à peu près cinq secondes.

7. Instrument chirurgical (10) de coupe et de fixation selon la revendication 1, dans lequel ledit dispositif de chronométrage (510) est soutenu dans ledit ensemble poignée (6).

8. Instrument chirurgical (10) de coupe et de fixation selon la revendication 1, dans lequel chacun desdits voyants lumineux (610) comprend une diode électroluminescente.

9. Instrument chirurgical (10) de coupe et de fixation selon la revendication 1, dans lequel ledit indicateur de durée (510) recommence à compter quand est appliqué ledit mouvement d'entraînement.

10. Instrument chirurgical (10) de coupe et de fixation selon la revendication 9, dans lequel ledit indicateur de durée (510) recommence à compter quand a été interrompue l'application dudit mouvement d'entraînement.

11. Procédé d'utilisation d'un ustensile chirurgical de coupe et de fixation, le procédé comprenant les opérations consistant à :
se procurer l'ustensile chirurgical (10) de coupe et de fixation selon l'une quelconque des revendications précédentes ;
stériliser au moins une partie de celui-ci ; et
ranger la partie stérile dans un récipient stérile.
